(19) Europäisches Patentamt
European Patent Office
Office européen des brevets

(11) EP 0 623 583 B1

(12) **EUROPÄISCHE PATENTSCHRIFT**

(45) Veröffentlichungstag und Bekanntmachung des Hinweises auf die Patenterteilung:
**19.03.1997 Patentblatt 1997/12**

(51) Int Cl.⁶: **C07C 68/00**, C07C 69/96

(21) Anmeldenummer: **94105984.2**

(22) Anmeldetag: **18.04.1994**

(54) **Verfahren zur Herstellung von Dialkylcarbonaten**

Process for the production of dialkyle carbonates

Procédé de préparation de carbonates de dialkyle

(84) Benannte Vertragsstaaten:
**BE CH DE ES FR GB IT LI NL**

(30) Priorität: **29.04.1993 DE 4314038**

(43) Veröffentlichungstag der Anmeldung:
**09.11.1994 Patentblatt 1994/45**

(73) Patentinhaber: **BAYER AG**
**51368 Leverkusen (DE)**

(72) Erfinder:
• **Jentsch, Joerg-Dietrich, Dr.**
**D-45468 Mülheim (DE)**

• **Klausener, Alexander, Dr.**
**D-50670 Köln (DE)**
• **Landscheidt, Heinz, Dr.**
**D-47057 Duisburg (DE)**
• **Wolters, Erich, Dr.**
**D-50939 Köln (DE)**
• **Zirngiebl, Eberhard, Dr.**
**D-51061 Köln (DE)**

(56) Entgegenhaltungen:
**EP-A- 0 503 091        EP-A- 0 503 618**
**EP-A- 0 559 001**

## Beschreibung

Die vorliegende Erfindung betrifft ein Verfahren zur Herstellung von Dialkylcarbonaten durch Umsetzung von Kohlenmonoxid (CO) mit Alkylnitriten in Gegenwart eines Katalysators aus der Reihe der Platinmetall-Halogenide auf einem Träger aus Metallphosphat mit sauren Zentren.

Dialkylcarbonate sind von allgemeiner chemischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs- und Acylierungsreagenzien. Schließlich haben sie große Bedeutung bei der Herstellung von anderen Carbonaten, von Urethanen und von Harnstoffen.

Es ist bekannt, Dialkylcarbonate durch Umsetzungen von Phosgen bzw. von Chlorameisensäurealkylestern mit Alkoholen herzustellen. Es besteht jedoch ein steigendes Interesse daran, den Einsatz des giftigen Phosgens bzw. der davon abgeleiteten Zwischenprodukte, wie der Chlorameisensäureester, durch andere Verfahren abzulösen. Neben Versuchen, Dialkylcarbonate durch Umsetzung von CO mit niederen Alkoholen zu erhalten, sind insbesondere Verfahren wichtig, in denen CO in der Gasphase mit Alkylnitrit an einem Platinmetall-Katalysator umgesetzt wird. Bei solchen Umsetzungen wird neben dem gewünschten Dialkylcarbonat häufig das Auftreten von Dialkyloxalat beobachtet.

So wird in der Zeitschrift für Katalytische Forschung (China), Vol. 10(1), S. 75-78 die Umsetzung von CO und Methylnitrit an einem $PdCl_2$-haltigen Aktivkohle-Katalysator beschrieben, wobei neben Dimethyloxalat größtenteils Dimethylcarbonat entsteht.

Ein Pd/Kohle-Katalysator wird auch in Chin. Sci. Bull. 34 (1989), 875-76 für die Herstellung von Dimethylcarbonat aus CO und Methylnitrit beschrieben.

Ferner ist aus EP 425 197 ein Verfahren bekannt, das gemäß seiner bevorzugten Ausführungsform zu Dialkylcarbonaten des Methanols bzw. Ethanols aus CO und Methyl- bzw. Ethylnitrit in der Gasphase an einem $PdCl_2$-Katalysator auf Aktivkohle führt. Die Selektivitäten zu den gewünschten niederen Dialkylcarbonaten erreichen gemäß dieser EP 425 197, Tabelle 1, Werte bis zu 94 %, jedoch werden als Nebenprodukte stets niedrige Dialkyloxalate und $CO_2$ beobachtet. Bei einer Nacharbeitung konnten darüber hinaus die angegebenen hohen Selektivitäten nur ungenügend reproduziert werden. Die Katalysatoren dieser EP 425 197 enthalten Zusätze von Chloriden unedler Metalle; ein beträchtlicher Zusatz von Chlorwasserstoff, nämlich eine Menge von 1 bis 50 Mol-%, bezogen auf das Platinmetall im Katalysator, wird dem System zugesetzt oder ein Teil des Katalysators muß aus dem Reaktor entnommen werden und einer Behandlung mit Chlorwasserstoff unterzogen werden.

In EP 501 507 wird die Verwendung von Zeolithen als Trägermaterial beschrieben. Dadurch wird zwar ein Teil der oben beschriebenen Nachteile umgangen, aber gleichzeitig tritt eine deutliche Verschlechterung der Selektivität bezüglich des eingesetzten Methylnitrits auf. So wird gemäß Beispiel 1 nur eine Selektivität von 79 %, basierend auf Methylnitrit, erzielt.

In EP 503 091 und EP 503 618 werden die Eigenschaften der Katalysatoren auf Aktivkohle-Basis durch weitere Zusätze, wie z.B. von Kupfer und Molybdän bzw. Kupfer, Molybdän und Kaliumfluorid, verbessert. Allerdings tritt auch hier eine Abnahme der Katalysator-Aktivität auf, die bei einem technischen Prozeß zu einem erheblichen Mehraufwand führt. Diese dadurch bedingten Nachteile, wie höherer Regelaufwand durch sich verändernde (fallende) Umsätze und Stillstandzeiten bei Austausch des desaktivierten Katalysators, machen sich besonders bei einem Kreisprozeß, bei dem das bei der Reaktion entstehende NO wieder zurückgeführt und in Methylnitrit umgewandelt wird, bemerkbar. Weiter nachteilig sind die geringen erzielten Umsätze an Methylnitrit. Dies ist ebenfalls im Zusammenhang mit dem oben erwähnten Kreisgasprozeß als nachteilig anzusehen.

In DE-OS 41 23 603 wird durch Anwendung eines Palladiumchlorid-Katalysators mit $\gamma$-$Al_2O_3$ als Trägermaterial eine hohe Selektivität, sowohl bezogen auf CO als auch auf Methylnitrit, bei gleichzeitig hohem Umsatz erzielt. Allerdings muß zur Aufrechterhaltung der katalytischen Aktivität Chlorwasserstoffgas in Mengen bis zu 1000 ppm dem Eduktgemisch zugesetzt werden. Dies kann zu Korrosionsproblemen führen.

Es wurde nun gefunden, daß die dargestellten Nachteile überwunden werden können, wenn Platinmetall-Trägerkatalysatoren eingesetzt werden, deren Träger Metallphosphate mit sauren Zentren sind. Metallphosphate werden erfindungsgemäß als Tabletten oder bindemittelhaltige Extrudate eingesetzt. Geeignete Bindemittel sind beispielsweise $SiO_2$, $Al_2O_3$ oder Tonmineralien. Die Bindemittelgehalte können in einem breiten Bereich, beispielsweise von 0,5 bis 99,5 Gew.-%, bezogen auf das Gesamtgewicht des Trägers, variiert werden.

Es wurde ein Verfahren zur Herstellung von Dialkylcarbonaten der Formel

$$O=C(OR)_2 \qquad (I),$$

worin

R für geradkettiges oder verzweigtes $C_1$-$C_4$-Alkyl steht,

durch Umsetzung von Kohlenmonoxid (CO) mit Alkylnithten der Formel

$$RONO \qquad (II),$$

worin

R die angegebene Bedeutung hat,

in Anwesenheit oder Abwesenheit eines Inertgases sowie in Anwesenheit oder Abwesenheit wesenheit des zugrundeliegenden Alkohols ROH sowie in Anwesenheit oder von NO an einem Platinmetall-Träger-Katalysator bei erhöhter Temperatur in kontinuierlicher Gasphasenreaktion gefunden, das dadurch gekennzeichnet ist, daß als Träger Metallphosphate mit sauren Zentren eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetallverbindung mit Hilfe von Halogenwasserstoff unter den Reaktionsbedingungen gebildet werden kann, und bei einem Volumenverhältnis von Nitrit : CO = 0,1 - 10:1, einem Druck von 0,5 bis 6 bar und einer Temperatur von 50 bis 150°C gearbeitet wird, wobei absatzweise oder kontinuierlich Halogenwasserstoff zugesetzt wird.

Für die erfindungsgemäße Verwendung als Katalysatorträger eignen sich u.a. folgende Metallphosphate: Saure Metallphosphate einschließlich saurer Metallmonohydrogenphosphate und saurer Metalldihydrogenphosphate aus Gruppe II A-Elementen, Gruppe III A-Elementen, Gruppe III B-Elementen, Gruppe IV B-Elementen, Gruppe V B-Elementen des Periodensystems der Elemente (Mendelejew), Seltenerd-Metalle der Atomnummern 58-71 und der Actiniden mit den Atomnummern 89-92 sowohl im Sinne chemisch einheitlicher Reinsubstanzen als auch im Gemisch. In bevorzugter Weise sind dies ein oder mehrere Metallphosphate, Metallmonohydrogenphosphate oder Metalldihydrogenphosphate aus der Gruppe von Magnesium, Calcium, Strontium, Barium, Titan, Zirkonium, Hafnium, Vanadium, Niob, Tantal, Bor, Aluminium, Gallium, Indium, Lanthan, Cer, Praseodym, Neodym, Samarium, Europium, Gadolinium, Terbium, Dysprosium, Holmium, Erbium, Thulium, Ytterbium, Lutetium und Uran.

Erfindungsgemäß verwendete Katalysatorträger können hergestellt werden durch Fällen des gewünschten sauren Metall-Phosphats, -Hydrogenphosphats oder -Dihydrogenphosphats, Waschen des Niederschlags zur Entfernung anorganischer Nebenprodukte und Trocknen.

Wahlweise können getrocknete Katalysatorträger weiter durch Extrudieren, Tablettieren, Zumischen weiterer Katalysatorträger wie $Al_2O_3$ oder $SiO_2$ und Calcinieren modifiziert werden.

Darstellung und Weiterverarbeitung sind dem Fachmann wohl bekannt und Stand der Technik.

Durch Anwendung oben beschriebener Präparationsmethoden erhält man im Reaktionsmedium unlösliche, poröse Feststoffe, die Lewis- und Brönstedt-Säure-Zentren enthalten. Ihre Zusammensetzung variiert aufgrund der vielfältigen Abhängigkeiten von den Herstellbedingungen, wie Temperatur, Konzentration und Natur der Reaktanden, Geschwindigkeit und Reihenfolge der Reaktanden-Einbringung, pH-Wert während der Präparation, Dauer der Fällung, Volumen und pH-Wert der Waschlösungen, Dauer und Temperatur von Trocknung und Calcinierung usw.. Diese wechselnde Zusammensetzung der Phosphate beeinflußt ihre Eignung als Katalysatorträger jedoch nur wenig.

Als besonders geeignet haben sich die sauren Phosphate, Hydrogenphosphate und Dihydrogenphosphate des Aluminiums, Vanadiums, Niobs, Yttriums, Lanthans, der Seltenerdmetalle (Atomnummern 58-71) und ihre Mischungen herausgestellt.

Die durch Verwendung des erfindungsgemäßen Katalysatorträgers erzielten Vorteile können gegebenenfalls durch Zusatz aus einer Verbindung von Eisen, Kupfer, Wismut, Kobalt, Nickel, Zinn, Molybdän, Wolfram sowie von Alkali- und Erdalkali-Metallen oder einem Gemisch mehrerer von ihnen weiter verbessert werden.

Die Reaktion im erfindungsgemäßen Verfahren erfolgt im Sinne der nachfolgenden Reaktionsgleichung:

$$CO + 2\ RONO \rightarrow O{:}C(OR)_2 + 2\ NO$$

R ist hierbei geradkettiges oder verzweigtes Alkyl mit 1-4 C-Atomen, beispielsweise Methyl, Ethyl, Propyl, Isopropyl, Butyl, Isobutyl, in bevorzugter Weise Methyl und Ethyl und in besonders bevorzugter Weise Methyl.

Grundsätzlich ist es möglich, von einem Gemisch verschiedener Alkylnitrite auszugehen, wobei jedoch auch ein Gemisch verschiedener Dialkylcarbonate und gegebenenfalls unsymmetrisch substituierte Dialkylcarbonate entstehen. Im Sinne einer einheitlichen Reaktion ist es daher bevorzugt, von nur einem Alkylnitrit auszugehen.

Während es grundsätzlich möglich ist, CO mit einem Alkylnitrit ohne weitere Gemischkomponente umzusetzen, beispielsweise wenn man sich in der Gemischzusammensetzung außerhalb der Explosionsgrenzen befindet, wird vielfach ein Inertgas zur Verdünnung der Reaktionspartner herangezogen. Inertgase sind beispielsweise Edelgase, Stickstoff und Kohlendioxid, bevorzugt Argon, Stickstoff oder Kohlendioxid, besonders bevorzugt Stickstoff und Kohlendioxid. Die Menge des Inertgases beträgt 20 bis 80 Vol.-%, bevorzugt 30 bis 70 Vol.-%, bezogen auf das gesamte in den Reaktor einzuführende Gasvolumen. Das Inertgas und gegebenenfalls darin enthaltene nicht umgesetzte Restmengen der Reaktionspartner kann recyclisiert werden.

Das Volumenverhältnis der Reaktionspartner Nitrit und CO unter sich beträgt 0,1 bis 10:1, bevorzugt 0,2 bis 4:1, besonders bevorzugt 0,3 bis 3:1.

Das umzusetzende Gasgemisch kann weiterhin geringe Mengen an Alkohol ROH, beispielsweise in einer Menge von 0 bis 10 Vol.-% und geringe Mengen an NO, beispielsweise in einer Menge von 0 bis 10 Vol.-%, beides bezogen auf das Gesamtvolumen des einzusetzenden Gasgemisches, enthalten. Solche Zusätze an ROH bzw. NO können etwa aus der Herstellung des Alkylnitrits stammen und beispielsweise mit diesem in das Reaktionsgasgemisch hereingebracht werden.

Der Katalysator für das erfindungsgemäße Verfahren wird auf Metallphosphaten als Träger aufgebracht. Seine Reaktivkomponente besteht im reaktionsbereiten Zustand aus einem Platinmetall-Halogenid oder einer Platinmetall-Halogenid enthaltenden Komplexverbindung. Solche Komplexverbindungen sind grundsätzlich bekannt und sind beispielsweise Alkalimetallchlorid-Komplexverbindungen, wie Lithium- oder Natrium-tetrachloropalladat, $Li_2[PdCl_4]$ bzw. $Na_2[PdCl_4]$.

Es hat sich weiterhin gezeigt, daß das Platinmetall-Halogenid bzw. die das Platinmetall-Halogenid enthaltende Komplexverbindung in situ im Reaktor aus metallischem Platinmetall oder einer halogenfreien Platinmetall-Verbindung unter den Reaktionsbedingungen, d.h. in Gegenwart des umzusetzenden Gasgemisches, mit Hilfe von Halogenwasserstoff gebildet werden kann. Der Reaktor kann demnach auch mit einem ansonsten vergleichbaren Katalysator gefüllt werden, der das Platinmetall zunächst in metallischer Form enthält oder der mit Hilfe einer halogenfreien Platinmetall-Verbindung hergestellt worden war. Solche halogenfreien Platinmetall-Verbindungen, die hierfür in Frage kommen, sind beispielsweise Platinmetall-nitrate, -propionate, -butyrate, -carbonate, -oxide, - hydroxide oder andere dem Fachmann geläufige.

Elemente der Gruppe der Platinmetalle im Sinne der Erfindung sind Palladium, Platin, Iridium, Ruthenium und Rhodium, bevorzugt Palladium, Ruthenium und Rhodium, besonders bevorzugt Palladium.

Halogenide im Sinne der Erfindung sind Fluorid, Chlorid, Bromid und Iodid, bevorzugt Chlorid und Bromid, besonders bevorzugt Chlorid.

Die Menge des Platinmetall-Halogenids oder der das Platinmetall-Halogenid enthaltenden Komplexverbindung im reaktionsbereiten Zustand beträgt 0,01 bis 8 Gew.-%, bevorzugt 0,05 bis 4 Gew.-%, gerechnet als Platinmetall und bezogen auf das Gesamtgewicht des Katalysators.

Die Herstellung eines erfindungsgemäß einzusetzenden Katalysators erfolgt nach Methoden, die dem Fachmann grundsätzlich bekannt sind. So kann der Träger mit einer Lösung einer oder mehrerer der genannten Platinmetall-Verbindungen getränkt oder besprüht werden. In gleicher Weise wird mit dem oder den genannten Zusätzen verfahren. Für den Fall, daß das Platinmetall als Metall oder in Form des Carbonats, Oxids oder Hydroxids auf dem Träger fixiert werden soll und erst im Reaktor in der beschriebenen Weise mit Hilfe von Halogenwasserstoff unter den Reaktionsbedingungen zum Platinmetall-Halogenid aktiviert werden soll, kann die aufgetragene Platinmetall-Verbindung in einer dem Fachmann bekannten Weise durch ein geeignetes Reduktionsmittel zum Metall reduziert werden oder durch ein geeignetes Fällungsmittel in das Carbonat, Oxid oder Hydroxid umgewandelt werden.

Es wurde ferner beobachtet, daß es zur Erzielung gleichmäßig hoher Selektivitäten an Dialkylcarbonat vorteilhaft ist, Halogenwasserstoff an den Katalysator im Verlauf seiner Einsatzzeit heranzubringen. Hierbei wurde grundsätzlich gefunden, daß bei größerer Menge an Halogenwasserstoff die Ausbeute steigt. So kann die Konzentration an Halogenwasserstoff (z.B. HCl), die mit dem Einsatzmaterial an den Katalysator gebracht wird, beispielsweise bis zu 1000 ppm betragen. Es wurde jedoch weiter beobachtet, daß diese Menge an Halogenwasserstoff wesentlich kleiner sein kann. So ist es lediglich erforderlich, die Menge des mit den Reaktionsprodukten ausgetragenen, der aktivierten Form des Katalysators entstammenden Halogenwasserstoffs zu ersetzen. Diese Menge kann analytisch bestimmt werden. Sie bewegt sich im allgemeinen in einem Bereich von 1 bis 2000 µg Halogenwasserstoff pro gebildetem g Dialkylcarbonat. Aus Gründen einer einfacheren Aufarbeitung kann es wünschenswert sein, wenig Halogenwasserstoff einzusetzen.

Halogenwasserstoff im Sinne der Erfindung ist Fluorwasserstoff, Chlorwasserstoff, Bromwasserstoff und Iodwasserstoff, bevorzugt Chlorwasserstoff und Bromwasserstoff, besonders bevorzugt Chlorwasserstoff.

Der Halogenwasserstoff kann als solcher gasförmig dem Reaktionsgemisch zudosiert werden. Er kann jedoch auch in gelöster Form in einem der im Reaktionsgemisch vorhandenen Stoffe eindosiert werden, so beispielsweise gelöst in dem dem Alkylnitrit zugrundeliegenden Alkohol.

Die genannten Katalysatoren können mit 700 bis 5000 l Gemisch der gasförmigen Reaktionspartner pro l Katalysator und pro Stunde belastet werden (GHSV).

Das erfindungsgemäße Verfahren wird bei einer Temperatur von 50 bis 150°C, bevorzugt 70 bis 120°C, besonders bevorzugt 70 bis 110°C, und einem Druck von 0,5 bis 10 bar, bevorzugt 0,8 - 7 bar, besonders bevorzugt 1 bis 6 bar, ganz besonders bevorzugt 1 bis 5 bar, durchgeführt.

Die Herstellung der erfindungsgemäß einzusetzenden Alkylnitrite erfolgt nach bekannten Verfahren, beispielsweise aus dem zugehörigen Alkohol und salpetriger Säure, die etwa in situ aus einem Alkalinitrit und einer Mineralsäure, wie Schwefelsäure, gebildet wird. Das im Verlaufe des erfindungsgemäßen Verfahrens gebildete Stickstoffmonoxid NO kann kontinuierlich mit Sauerstoff und neuem Alkohol zu Alkylnitrit regeneriert werden (DE-OS 38 34 065) und gemeinsam mit nicht umgesetzten Reaktionspartnern recyclisiert werden.

### Beispiele

Definitionen

Die Raumzeitausbeute (Space Time Yield STY) in [g/l.h] und für Dimethylcarbonat in den Beispielen berechnet sich nach

$$\frac{m_{DMC}}{V_{Kat}.t},$$

wobei

$m_{DMC}$ die Menge gebildetes Dimethylcarbonat (DMC),

$V_{Kat}$ das Katalysatorvolumen und t die Zeit bedeuten.

Die Selektivität S (%) wird berechnet nach

$$S = \frac{n_{DMC}}{n_{DMC} + 2 x n_{ODME} + n_{AME} + n_{FDA}} \; x \; 100 \; [\%]$$

wobei

$n_{DMC}$ = Stoffmenge Dimethylcarbonat
$n_{ODME}$ = Stoffmenge Oxalsäuredimethylester
$n_{AME}$ = Stoffmenge Ameisensäuremethylester
$n_{FDA}$ = Stoffmenge Formaldehyddimethylacetal

bedeuten.

**Beispiel 1** (Herstellung eines Katalysators)

Zu einer Lösung von 650 g (1,5 Mol) Lanthannitrat-Hexahydrat in 4,5 l entionisiertem Wasser wurden bei +50°C innerhalb von 60 Minuten 2,25 l einer Lösung, die 396 g (3 Mol) Ammoniumhydrogenphosphat enthielt, getropft. Der pH-Wert der entstandenen weißen Suspension wurde mit wäßrigem Ammoniak auf pH 6 gestellt. Man rührte 30 Minuten nach, saugte das Produkt ab und wusch auf der Nutsche nitratfrei.

Einer 18stündigen Trocknung bei 120°C schloß sich ein Kalzinierungsschritt bei 500°C (16 h) sowie eine Tablettierung an.

100 ml des so erhaltenen Katalysatorträgers wurden mit einer wäßrigen $Li_2PdCl_4$-Lösung getränkt und das Produkt bei 80°C im Vakuum (29 Torr) getrocknet. Der Katalysator enthielt dann 8 g Pd/l.

**Beispiel 2** (Herstellung eines Katalysators)

Zu einer Lösung von 900 g (2,1 Mol) Lanthannitrat-Hexahydrat in 3 l entionisiertem Wasser wurden bei 50°C 3 l verdünnte Phosphorsäure, entsprechend 408 g = 4,16 Mol 100 % Phosphorsäure, innerhalb von 15 min getropft. Man stellte die Suspension mit wäßrigem Ammoniak auf pH = 6 und ließ 3 h bei 50°C nachrühren.

Nach Waschen auf der Nutsche wurde das Produkt 12 h bei 120°C getrocknet und tablettiert.

100 ml des so erhaltenen Katalysatorträgers wurden mit einer wäßrigen $Li_2PdCl_4$-Lösung getränkt und das Produkt bei 80°C im Vakuum (29 Torr) getrocknet. Der Katalysator enthielt dann 8 g Pd/l.

**Beispiel 3** (Verfahrensdarstellung)

In einem vertikal aufgestellten Glasrohr (Länge 50 cm, Durchmesser 4 cm) wurden zwischen einer Füllung aus Raschig-Ringen 20 ml des Katalysators aus Beispiel 1 gebracht.

Das Glasrohr wurde auf 90°C erhitzt, und ein Gasgemisch aus 55 % $N_2$, 20 % Methylnitrit (MeONO), 20 % CO und 5 % Methanol (MeOH) wurde durchgeleitet. Dem Gasgemisch wurden 50 ppm HCl (Volumen) zugesetzt. Die Raumgeschwindigkeit betrug 1000 l/l·h.

Das dem Reaktor entströmende Gas wurde auf 5°C gekühlt und die erhaltene kondensierte Phase mittels Gaschromatographie untersucht.

Die nicht kondensierten Produkte wurden mittels IR-Spektroskopie und Massenspektroskopie erfaßt.

Dimethylcarbonat wurde nach 2 h mit STY von 140 g/l h und S = 99 % gebildet.

Auch nach 10 h betrug STY 140 g/l h und S 99 %.

**Beispiel 4**

In Beispiel 4 war die Durchführung wie in Beispiel 3. Es wurden 10 ml Katalysator aus Beispiel 2 eingesetzt.

Dimethylcarbonat wurde nach 2 h mit STY von 145 g/l h und S = 99 % gebildet.

Auch nach 10 h betrug STY 145 g/l h und S 99%.

**Patentansprüche**

1. Verfahren zur Herstellung von Dialkylcarbonaten der Formel

$$O = C(OR)_2,$$

worin

R für geradkettiges oder verzweigtes $C_1$-$C_4$-Alkyl, bevorzugt Methyl oder Ethyl, besonders bevorzugt Methyl, steht,

durch Umsetzung von Kohlenmonoxid (CO) mit Alkylnitriten der Formel

$$RONO,$$

worin

R die angegebene Bedeutung hat,

in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion, dadurch gekennzeichnet, daß als Träger Metallphosphate mit sauren Zentren eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetallverbindung mit Hilfe von Halogenwasserstoff unter den Reaktionsbedingungen gebildet werden kann, und bei einem Volumenverhältnis von Nitrit:CO=0,1-10:1, bevorzugt 0,2-4:1, besonders bevorzugt 0,3-3:1, einem Druck von 0,5 bis 10 bar, bevorzugt 0,8 bis 7 bar, besonders bevorzugt 1 bis 6 bar, ganz besonders bevorzugt 1 bis 5 bar, und einer Temperatur von 50 bis 150°C, bevorzugt 70 bis 120°C, besonders bevorzugt 70 bis 110°C gearbeitet wird, wobei absatzweise oder kontinuierlich Halogenwasserstoff zugesetzt wird.

2. Verfahren nach Anspruch 1, dadurch gekennzeichnet, daß als Träger ein saures Metallphosphat, saures Metallmonohydrogenphosphat oder saures Metalldihydrogenphosphat eines Elementes der Gruppe IIA, IIIA, IIIB, IV B, VB des Periodensystems der Elemente (Mendelejew), eines Seltenerd-Metalls der Atomnummern 58-71 oder der Actimiden der Atomnummern 89-92 oder ein Gemisch mehrerer von ihnen eingesetzt wird.

3. Verfahren nach Anspruch 2, dadurch gekennzeichnet, daß ein Metallphosphat, Metallmonohydrogenphosphat oder Metalldihydrogenphosphate aus der Gruppe von Magnesium, Calcium, Strontium, Barium, Titan, Zirkonium, Hafnium, Vanadium, Niob, Tantal, Bor, Aluminium, Gallium, Indium, Lanthan, Cer, Praseodym, Neodym, Samarium, Europium, Gadolinium, Terbium, Dysprosium, Holmium, Erbium, Thulium, Ytterbium, Lutetium, Uran im Sinne chemisch einheitlicher Reinsubstanzen wie auch im Gemisch eingesetzt werden.

4. Verfahren nach Anspruch 3, dadurch gekennzeichnet, daß ein Phosphat, Hydrogenphosphat oder Dihydrogenphosphat von Aluminium, Vanadium, Niob, Yttrium, Lanthan oder den Seltenerd-Metallen mit den Atomnummern 58-71 oder ein Gemisch mehrerer von ihnen eingesetzt wird.

5. Verfahren nach Anspruch 1, dadurch gekennzeichnet, daß dem Katalysator eine Verbindung von Eisen, Kupfer, Wismut, Kobalt, Nickel, Zinn, Molybdän, Wolfram oder von Alkali- oder Erdalkalimetallen oder ein Gemisch mehrerer von ihnen zugesetzt werden.

6. Verfahren nach Anspruch 1, dadurch gekennzeichnet, daß als Platinmetall eines oder mehrere aus der Gruppe von Palladium, Platin, Iridium, Ruthenium und Rhodium, bevorzugt aus der Gruppe von Palladium, Ruthenium und Rhodium, besonders bevorzugt Palladium eingesetzt wird (werden).

7. Verfahren nach Anspruch 1, dadurch gekennzeichnet, daß als Halogenid eines Platinmetalls eines oder mehrere aus der Gruppe der einfachen oder komplexen Fluoride, Chloride, Bromide und Iodide, bevorzugt aus der Gruppe der Chloride und Bromide, besonders bevorzugt der Chloride eingesetzt wird (werden).

8. Verfahren nach Anspruch 1, dadurch gekennzeichnet, daß in Anwesenheit eines Inertgases gearbeitet wird, wobei das Inertgas 20 bis 80 Vol.-%, bevorzugt 30 bis 70 Vol.-%, des gesamten Gasvolumens beträgt.

9. Verfahren nach Anspruch 1, dadurch gekennzeichnet, daß bei einer Katalysatorbelastung von 700 bis 5000 l Gemisch der gasförmigen Reaktionspartner pro Stunde und pro l Katalysator gearbeitet wird.

10. Verfahren nach Anspruch 1, dadurch gekennzeichnet, daß der Katalysator im reaktionsbereiten Zustand ein Platinmetallhalogenid oder eine ein Platinmetallhalogenid enthaltende Komplexverbindung in einer Menge von 0,01 bis 8 Gew.-%, bevorzugt 0,05 bis 4 Gew.-%, gerechnet als Platinmetall und bezogen auf das Gesamtgewicht des Katalysators, enthält.

## Claims

1. Process for preparing dialkyl carbonates of the formula

$$O = C(OR)_2,$$

in which

R represents a straight-chain or branched $C_1$-$C_4$-alkyl, preferably methyl or ethyl, particularly preferably methyl,

by reaction of carbon monoxide (CO) with alkyl nitrites of the formula

$$RONO,$$

in which

R is as defined above,

in the presence or absence of an inert gas and also in the presence or absence of the alcohol ROH on which it is based and also in the presence or absence of NO over a supported platinum metal catalyst at elevated temperature in a continuous gasphase reaction, characterized in that metal phosphates having acid centres are used as support, the platinum metal is present in the form of a halide or a halide-containing complex, which may each be formed in situ in the process reactor from the platinum metal or a halogen-free platinum metal compound with the aid of hydrogen halide under the reaction conditions, the reaction is carried out with a volume ratio of nitrite : CO = 0.1 - 10:1, preferably 0.2-4:1, particularly preferably 0.3-3:1, a pressure from 0.5 to 10 bar, preferably from 0.8 to 7 bar, particularly preferably from 1 to 6 bar, most preferably from 1 to 5 bar, and a temperature from 50 to 150°C, preferably from 70 to 120°C, particularly preferably from 70 to 110°C, and hydrogen halide is added stepwise or continuously.

2. Process according to Claim 1, characterized in that the support used is an acid metal phosphate, acid metal monohydrogen phosphate or acid metal dihydrogen phosphate of an element of Group IIA, IIIA, IIIB, IVB or VB of the Periodic Table of the Elements (Mendeleev), of a rare-earth metal of atomic numbers 58-71 or of the actinides of atomic numbers 89-92 or a mixture of a plurality thereof.

3. Process according to Claim 2, characterized in that the metal phosphate, metal monohydrogen phosphate or metal dihydrogen phosphate used is selected from the group consisting of magnesium, calcium, strontium, barium, titanium, zirconium, hafnium, vanadium, niobium, tantalum, boron, aluminium, gallium, indium, lanthanum, cerium, praseodymium, neodymium, samarium, europium, gadolinium, terbium, dysprosium, holmium, erbium, thulium, ytterbium, lutetium and uranium, as chemically uniform pure substances or as mixtures.

4. Process according to Claim 3, characterized in that a phosphate, hydrogen phosphate or dihydrogen phosphate of aluminium, vanadium, niobium, yttrium, lanthanum or of the rare-earth metals having atomic numbers 58-71 or a mixture of a plurality thereof is used.

5. Process according to Claim 1, characterized in that a compound of iron, copper, bismuth, cobalt, nickel, tin, molybdenum, tungsten or of alkali or alkaline-earth metals or a mixture of a plurality thereof is added to the catalyst.

6. Process according to Claim 1, characterized in that the platinum metal(s) used is/are one or more selected from the group consisting of palladium, platinum, iridium, ruthenium and rhodium, preferably from the group consisting of palladium, ruthenium and rhodium, particularly preferably palladium.

7. Process according to Claim 1, characterized in that the platinum metal halide(s) used is/are one or more selected from the group consisting of simple or complex fluorides, chlorides, bromides and iodides, preferably from the group consisting of chlorides and bromides, particularly preferably of chlorides.

8. Process according to Claim 1, characterized in that the reaction is carried out in the presence of an inert gas, the inert gas comprising from 20 to 80% by volume, preferably from 30 to 70% by volume, of the total gas volume.

9. Process according to Claim 1, characterized in that the reaction is carried out at a space velocity over the catalyst from 700 to 5000 l of the mixture of the gaseous reactants per hour per 1 of catalyst.

10. Process according to Claim 1, characterized in that in the active state the catalyst contains from 0.01 to 8% by weight, preferably from 0.05 to 4% by weight, of a platinum metal halide or of a complex containing a platinum metal halide, calculated as platinum metal and based on the total weight of the catalyst.

**Revendications**

1. Procédé pour la préparation de dialkylcarbonates répondant à la formule

$$O=C(OR)_2$$

dans laquelle

R représente un groupe alkyle en $C_1$-$C_4$ à chaîne droite ou ramifiée, de préférence un groupe méthyle ou un groupe éthyle, de manière particulièrement préférée un groupe méthyle,

par mise en réaction de monoxyde de carbone (CO) avec des nitrites d'alkyle répondant à la formule

$$RONO$$

dans laquelle

R a la signification indiquée,

en présence ou en l'absence d'un gaz inerte, ainsi qu'en présence ou en l'absence de l'alcool de départ ROH, ainsi qu'en présence ou en l'absence de NO, sur un catalyseur de métal du platine sur un support à température élevée dans une réaction en continu en phase gazeuse, caractérisé en ce que, comme support, on met en oeuvre des phosphates métalliques possédant des centres acides, le métal du platine étant présent sous forme d'un halogénure ou d'un composé complexe contenant un halogénure, dans lequel on peut former l'halogénure de métal du platine ou le complexe halogéné contenant un métal du platine in situ dans le réacteur du procédé à partir du métal du platine ou d'un composé de métal du platine exempt d'halogène à l'aide d'acide halogénhydrique dans les conditions réactionnelles, et on travaille dans un rapport volumique nitrite:$CO = 0,1$-$10:1$, de préférence $0,2$-$4:1$, de manière particulièrement préférée $0,3$-$3:1$, sous une pression de 0,5 à 10 bar, de préférence de 0,8 à 7 bar, de manière particulièrement préférée de 1 à 6 bar, de manière tout particulièrement préférée de 1 à 5 bar, et à une température de 50 à 150°C, de préférence de 70 à 120°C, de manière particulièrement préférée de 70 à 110°C, dans lequel on ajoute l'acide halogénhydrique en discontinu ou en continu.

2. Procédé selon la revendication 1, caractérisé en ce que, comme support, on met en oeuvre un phosphate métallique acide, un monohydrogénophosphate métallique acide ou encore un dihydrogénophosphate métallique acide d'un élément des groupes IIA, IIIA, IIIB, IVB, VB du système périodique des éléments (Mendelejew), d'un métal des terres rares des nombres atomiques 58-71 ou des actinides des nombres atomiques 89-92 ou encore un mélange de plusieurs d'entre eux.

3. Procédé selon la revendication 2, caractérisé en ce qu'on met en oeuvre un phosphate métallique, un monohydrogénophosphate métallique ou des dihydrogénophosphates métalliques choisis parmi le groupe comprenant le magnésium, le calcium, le strontium, le baryum, le titane, le zirconium, le hafnium, le vanadium, le niobium, le tantale, le bore, l'aluminium, le gallium, l'indium, le lanthane, le cérium, le praséodyme, le néodyme, le samarium, l'europium, le gadolinium, le terbium, le dysprosium, l'holmium, l'erbium, le thulium, l'ytterbium, le lutécium, l'uranium dans le sens de substances pures homogènes du point de vue chimique, de même qu'en mélange.

4. Procédé selon la revendication 3, caractérisé en ce qu'on met en oeuvre un phosphate, un hydrogénophosphate ou un dihydrogénophosphate de l'aluminium, du vanadium, du niobium, de l'yttrium, du lanthane ou des métaux des terres rares possédant les nombres atomiques 58-71 ou encore un mélange de plusieurs d'entre eux.

5. Procédé selon la revendication 1, caractérisé en ce qu'on ajoute au catalyseur un composé du fer, du cuivre, du bismuth, du cobalt, du nickel, de l'étain, du molybdène, du tungstène ou de métaux alcalins ou alcalino-terreux ou encore un mélange de plusieurs d'entre eux.

6. Procédé selon la revendication 1, caractérisé en ce que, comme métal du platine, on met en oeuvre un ou plusieurs métaux choisis parmi le groupe comprenant le palladium, le platine, l'iridium, le ruthénium et le rhodium, de préférence choisis parmi le groupe comprenant le palladium, le ruthénium et le rhodium, de manière particulièrement préférée le palladium.

7. Procédé selon la revendication 1, caractérisé en ce que, comme halogénure d'un métal du platine, on met en oeuvre un ou plusieurs halogénures choisis parmi le groupe comprenant les fluorures, les chlorures, les bromures et les iodures simples ou complexes, de préférence choisis parmi le groupe comprenant les chlorures et les bromures, de manière particulièrement préférée les chlorures.

8. Procédé selon la revendication 1, caractérisé en ce qu'on travaille en présence d'un gaz inerte, le gaz inerte représentant de 20 à 80% en volume, de préférence de 30 à 70% en volume du volume de gaz total.

9. Procédé selon la revendication 1, caractérisé en ce qu'on travaille à une charge de catalyseur de 700 à 5000 1 de mélange des partenaires réactionnels gazeux par heure et par litre de catalyseur.

10. Procédé selon la revendication 1, caractérisé en ce que le catalyseur, à l'état prêt pour la réaction, contient un halogénure de métal du platine ou un composé complexe contenant un halogénure de métal du platine en une quantité de 0,01 à 8% en poids, de préférence de 0,05 à 4% en poids, calculés comme métal du platine et rapportés au poids total du catalyseur.